# EUROPÄISCHE PATENTANMELDUNG

(11) **EP 0 876 792 A1**
(43) Veröffentlichungstag der Anmeldung: **11.11.1998**
(21) Anmeldenummer: 97107500.7
(22) Anmeldetag: 07.05.1997
(51) Int. Cl.: A61B 5/14, A61L 33/00, A61M 5/315, A61M 5/14

(54) **Einfache Vorrichtung zur Einsparung von Einmalspritzen und Blut bei der Blutprobenentnahme aus Kathetern**

(71) Anmelder: Olivier, Lucien Christoph, Dr. med., 47228 Duisburg (DE)
(72) Erfinder: Olivier, Lucien Christoph, Dr. med., 47228 Duisburg (DE)

(57) **Zusammenfassung**

Bei der Entnahme von Laborblutproben aus arteriellen und venösen Kathetern wird stets eine gewisse Menge Blut zuvor hieraus aufgezogen und samt Einmalspritze verworfen, da das Probenblut ansonsten mit laufenden Infusionen vermischt ist und Meßfehler entstehen. Dieses Blut und die notwendigen Einmalspritzen sollen durch eine kostengünstige Vorrichtung gespart werden.

Die Idee ist, dieses Blut innerhalb eines geschlossenen Systemes zeitweilig steril und möglichst vor Veränderungen geschützt aufzubewahren und nach der eigentlichen Blutprobengewinnung wieder dem Patienten zurückzugeben. Dies kann durch einem langen Infusionsschlauch, der vorzugsweise aus gerinnungshemmenden Material erzeugt wird, geleistet werden. Dieser wird zwischen zwei Abnahmepunkte vor den Katheter geschaltet. Eine wichtige - auf andere Spritzensysteme übertragbare - Neuerung ist eine durch zwei ganzradiäre Führungen ihres Stempels auch unter wiederholter Nutzung steril bleibende Einmalspritze. Ihr Stempel wird vorne und hinten mit einem Abnahmepunkt versehen in die Infusionslinie eingeschaltet und bei Bedarf zur Aspiration aus dem Katheter verwendet. Das Blut kann dann zurückgegeben werden.

## Beschreibung

Die Erfindung betrifft einerseits ein bewußt sehr preiswert herstellbares System zur Einsparung von Blut bei der Laborblutentnahme andererseits eine ebenso kostengünstige einfache Veränderung von Einwegspritzen, sodaß diese am gleichen Patienten bakteriell kontaminationsgeschützt wiederverwendet werden können.

Auf Intensiv- und Normalstationen wird bei der Laborblutentnahme an zentralen Venen - aber auch arteriellen Kathetern stets eine gewisse Menge Blut zuvor aspiriert und mitsamt der Spritze verworfen. Auf diese Weise erhält man repräsentatives Blut des Patienten im Katheter, das ja im Katheter unter laufender Infusion mit dieser vermischt wäre. Einerseits werden so größere Mengen Spritzen regelmäßig weggeworfen, andererseits erleidet der Patient eine oft transfusionsbedürftige diagnostische Anämie. Dieses Thema ist auf Intensivstationen besonders offensichtlich, da es hier zu erheblichen iatrogenen Blutverlusten kommen kann. Nicht zuletzt aufgrund der aktuellen AIDS-Diskussion besteht allgemein ein großes Interesse daran, vermeidbare Fremdblutübertragungen z.B. eben durch unnötigen Eigenblutverlust dem Patienten zu ersparen. Die durch diese Vorgchensweise verworfene Blutmenge beträgt pro Tag bei einem beatmeten intensivstationären Patienten 20 bis 40 ml. D.h., über einen durchschnittlichen Zeitraum von 10 Tagen auf der Intensivstation geben zwischen 200 und 400 ml Blut verloren, ohne daß hieraus irgendein Laborwert o.ä.bestimmt worden wäre.

Ein anderes mit diesem Problem gekoppeltes Thema ist die ebenfalls aktuelle Diskussion der Müllvermeidung. Bei der diagnostischen Blutprobenentnahme wird nämlich - wie erwähnt - das zuvor aufgezogene nicht repräsentative Blut mitsamt der Spritze verworfen. Bei stündlichen Blutgasbestimmungen sind dies bis zu 24 Einmalspritzen (2ml), bei venöser Blutentnahme im Schnitt zwei größere (5 bis 10 ml) pro Tag. Das Verwerfen des Blutes erfolgt, weil der Arzt oder die Schwester mit einer Aktivierung der Gerinnung des in der Spritze aufgezogenen Blutes rechnen muß. Die Sorge ist, beim Zurückspritzen dieses Blutes frische Thromben in den Patienten zu injezieren. Dies könnte bei arterieller Gabe periphere Embolien zur Folge haben. Venös könnte ein Thrombus als lungenembolie verschleppt werden oder bei Übertritt in das linke Herz durch eine vorhandene Shuntverbindung sogar via Arterie ins Gehirn gelangen.

Bei einer arteriellen Blutentnahme Erwachsener werden stets 2ml Blut verworfen. Das macht bei durchschnittlich 12 arteriellen Blutproben pro Tag für die Blutgasbestimmung unter Beatmung 24 ml. D.h., in 12 Tagen Intensivbehandlung verliert ein Patient fast 300 ml Blut allein auf diesem Wege. Dies entspricht dem Inhalt einer Blutkonserve, die mehr als 250 DM kostet. Bei der venösen Blutentnahme über zentrale Venenkatheter werden für die Tageslaborroutine mindestens 10 ml Blut verworfen. Erfolgt - wie üblich - abends eine weitere Kontrolle, werden noch einmal 10 ml abgenommen. Könnte man die Menge des verworfenen Blutes auf Null reduzieren, könnten innnerhalb von 12 Tagen intensivstationärer Therapie durchschnittlich 500 bis 600 ml Vollblut gespart werden, d.h. sichere 500 DM. Je nach örtlichen Begebenheiten oft auch viel mehr.

Die Lösung dieses Problemes ist folgende: In Klammern ○ erfindungsgemäße Neuerungen, aus denen Patentansprüche abgeleitet werden

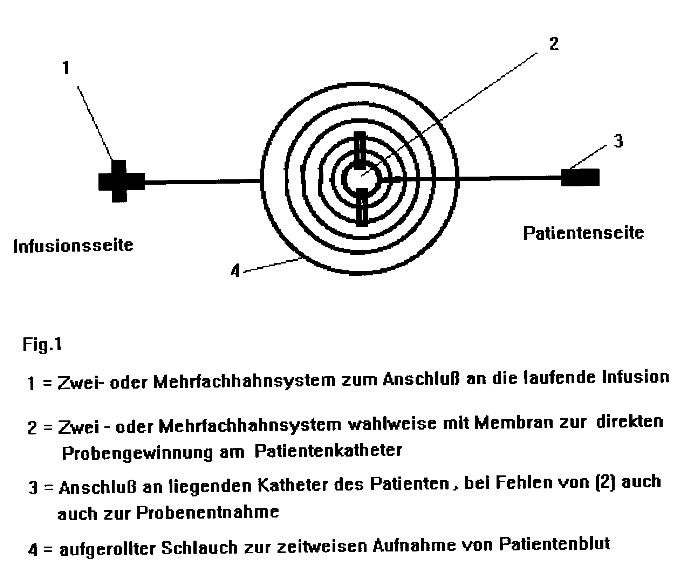

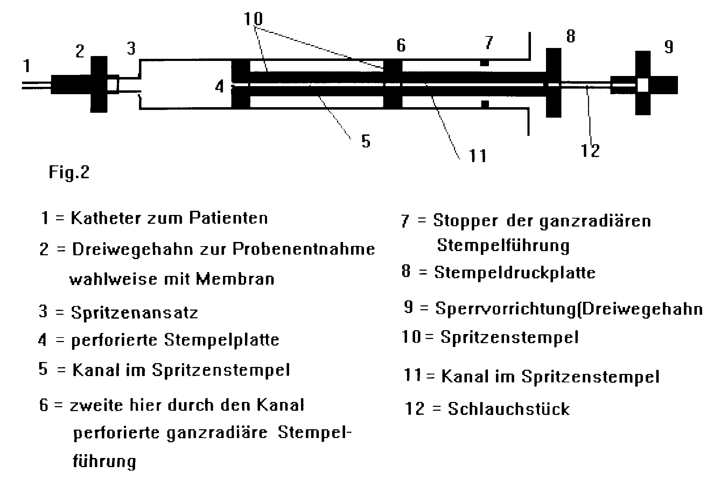

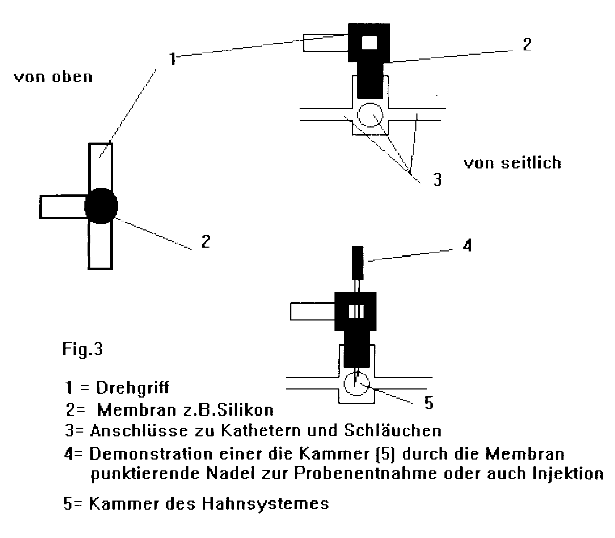

Die Grundidee ist nun, diesen Nachteilen einer Reinjektion dieses Blutes dadurch zu begegnen, daß das vor der eigentlichen Probengewinnung aufgezogene Blut erfindungsgemäß zeitweise in einem sterilen, mehrfach am Tage wiederverwendbaren Reservoir aufbewahrt wird, dessen innere Oberfläche nur minimalste Gerinnungsaktivierungen oder keine hervorruft (1). Diese Materialien sind bekannt und werden in Form von Polyurethan oder auch als an Heparin gebundene Werkstoffe angeboten. Heparin hemmt in einer Dosierung von 0.25 Units pro Milliliter Blut zuverlässig die Gerinnung. Andere Materialien sind durch spezielle Verfahren mit z.B. Aprotinin chemisch dotierte preiswerte Kunststoffe wie Polyethylen. Auch Aprotinin hemmt die Gerinnungskaskade. Andere Materialien sind in der Entwicklung arbeiten jedoch nach dem gleichen Prinzip der minimalen Gerinnungsaktivierung durch Oberflächenkontakt. Normalerweise ist z.B. ein zentraler Venenkatheter über mehrere Dreiwegeinfusionshähne mit den laufenden Infusionen für den Patienten verbunden. Schließt man jedoch zunächst einen sehr langen Schlauch an diesen Katheter an (2) und verbindet diesen dann erst mit den laufenden Infusionen (3), kann man vor der Entnahme der Blutproben zunächst hinter diesem Schlauch mit einer Spritze diesen von Infusionlösungen evakuieren (4) und so im eigentlichen Katheter repräsentatives Blut aufziehen,während das sonst verworfene Blut im überlangen Schlauchsystem steht (5). Über z.B. einen Dreiwegehahn (6) kann jetzt die erforderliche Laborblutprobe vor diesem überlangen Schlauchsystem aus dem eigentlichen Katheter gewonnen werden. Danach werden alle Hähne wieder geöffnet.Die laufende Infusionslösung wäscht das im überlangen Schlauchsystem stehende Blut in den Patienten zurück. Die Spritze, in der die Infusionslösung aus dem überlangen Schlauchsystem aufgezogen wurde,wird den Inhalt z.B. verwerfend (7) entleert und kann wiederverwendet (8) werden.Vorzugsweise wird das überlange Schlauchsystem gerollt verarbeitet (9), sodaß ein handliches Zubehör (10) zur Infusionstherapie entsteht. Die Anschlüsse erfolgen im bekannten Luersystem. Die eigentlichen Blutproben können an einem speziellen Zwei- oder Mehrwegehahnsystem (11) gewonnen werden Fig.3. Diese Entnahmestelle kann auch zunächst ein einfacher Dreiwegehahn (12) sein. Das spezielle Hahnsystem (Fig.3) ermöglicht jedoch nach der Aspiration des Blutes in das überlange Schlauchsystem, diesen Weg zeitweise zu blockieren,um dann senkrecht von oben (13) den Ansatz der eigentlichen Probenspritzen einzuführen und direkt das gewünschte Probenblut aus dem Katheter aufzuziehen. Dies entspricht einer weiteren erfindungsgemäßen Neuerung.Ebenso wäre es neu und damit erfindungsgemäß, diesen Zweiwegehahn nach oben mit einer eingepreßten Silikonmembran(14) - ähnlich den bekannten Portinfusionssystemen - zuversehen (Siehe hierzu:Deutsche Medizinische Wochenschrift 114 (1989) S.655 bis 656).

Man erhielte so eine einfach zu desinfizierende glatte Fläche (15), durch die hindurch man mit einem konventionellen Probensystem mit der Punktionsnadel eine Blutprobe ziehen könnte. Dies könnte aus infektiologischer Sicht z.B. beim immunsupprimierten Patienten gewünscht sein. Durch die Verwendung eines solchen "Portdreiwegehahnes"(17) bliebe die Option, Blut ohne Nadel abnehmen zu können, jedoch erhalten.

Eine zweite mögliche Lösung im Sinne von (1) ist eine Spritze aus dem o.g. Materialien zu fertigen,die mehrmals am Tage so wie sonst üblich benutzt wird (18), um den Katheter mit repräsentativem Blut zu füllen. Aufgrund der mechanischen Bewegung des Spritzenkolbens könnte es beim mehrfachen Gebrauch der Spritze jedoch zu Verlusten der gerinnungshemmenden Wirkung ihrer inneren Oberfläche kommen. Üblicherweise wird eine Einmalspritze jedoch nicht am Katheter belassen, sodaß es zu einer bakteriellen Kontamination der Spritzenanschlüsse kommt, was zum vorzeitigen Verwerfen der Spritze zwänge. Ihr Hauptnachteil ist jedoch, daß etwaige doch gebildete Thromben durch den hohen Druck, der mit einer Spritze erzeugt werden kann (ca.3 bar), in den Patienten gepreßt werden. Ein Schlauchsystem wie oben erlaubt hingegen die Beobachtung des langsamen Rücklaufes ohne Drucküberhöhung.

Ein weiterer Nachteil einer einfachen Spritze aus gerinnungshemmendem Kunststoff ist, daß sie nicht in der Längsachse des eingeführten Katheters zu liegen kommt. Stets steckt sie senkrecht an einem der Dreiwegehähne. Würde man die Infusionslösungen im unbenutzten Zustand durch diese Spritze laufen lassen können (19), läge sie in der Längsachse des Katheters (20). Dies ist dadurch zu erreichen, indem man den Spritzenstempel mit einem Kanal versieht (21), durch den die Infusionslösung fließt. Durch eine einfache - entweder im Spritzenstempel selbst gelegene (22) oder vor diesem an der Infusion (23) befindliche - Sperrvorrichtung (24) kann der Infusionsfluß unterbrochen werden. Die Spritze wird jetzt benutzt, um Blut und Infusionen zurückzusaugen (25), um den an ihr angeschlossen Patientenkatheter mit repräsentativem Blut zu füllen.

Die Blutprobenentnahme erfolgt dann vor der Spritze an einem Dreiwegehahn (26). Auch die Integration eines "Portdreiwegehahnes" wäre hier zur Probengewinnung sinnvoll (27). Das zeitweise aspirierte Blut wird wieder reinjeziert und der Infusionsfluß wird wieder durch den Spritzenstempel freigegeben.

Um bakterielle Kontaminationen der Spritze, die ja hinten offen ist, zu vermeiden,wird ihr Stempel mittig mit einer zweiten ganzradiären Führung (28) versehen. Die Stempelbewegung erfolgt nur bis zu diesem. Die beiden ganzradiären Führungen sind so angeordnet, daß sie zu keinem Zeitpunkt der Injektions - oder Aspirationsbewegung der Spritze eine gemeinsame Stelle im Spritzenzylinder berühren (31). Die Spritze ist so auch bei mehrfacher Verwendung kontaminationsgeschützt. Diese wesentliche Neuerung kann auf vorhandene andere Spritzensysteme z.B. sogenannte Perfusorspritzen übertragen werden um so in erheblichen Umfang durch ihre Wiederverwendbarkeit am gleichen Patienten kostensenkend und müllvermeidend eingesetzt zu werden (30).

## Patentansprüche

1. Steriles Reservoir aus gerinnungshemmendem Kunststoff zur zeitweisen Aufnahme von Patientenblut, das dann reinjeziert wird.

2. Schlauchsystem aus gerinnungshemmendem Kunststoff (Fig.1) verbunden mit je einein Dreiwegehahn (z.B.Luersystem) am Anfang und Ende des Schlauches zur Konnektion in ein vorhandenes Infusionssystem bei liegenden Kathetern Fig.1/1 und Fig.1/3.

3. Schlauchsystem wie zu 2. zur Platzeinsparung aufgerollt Fig.1/4.

4. Zwei- oder Mehrhahnsystem,wie 4., das jedoch eine zusätzliche Membran enthält durch die hindurch per Punktion Proben innerhalb des Hahnsystemes gezogen werden können Fig.1/5 bzw. Fig.3.

5. Positionierung eines weiteren Zwei- oder Mehrfachhahnes wie in 2. oder 4. oder 5. in der Mitte des aufgerollten Schlauches als eigentlichem Probenentnahmehahn Fig.1/5.

6. Einmalspritze aus gerinnungshemmendem Kunststoff zur Reinjektion von Blut bei der- Blutprobenentnahme.

7. Einmalspritze mit doppelter ganzradiärer Führung Fig.2/6 des Spritzenstempels und Stoppvorrichtung Fig.2/7 zur kontaminationsgeschützten Wiederverwendung derselben: auch als auf andere Spritzensysteme übertragbare Modifikation.

8. Einmalspritze mit längsgestelltem integrierten Kanal Fig.2/5 des Spritzenstempels zur Durchleitung von Infusionen.

9. Sperrvorrichtung des Kanales in 8. Fig.2/9 zur Nutzung der Spritze zwecks Aspiration von Blut.

10. Konnektion dieses in 1. bis 10. genannten Systemes Fig.1 und Fig.2 mit einem Zwei-oder Mehrfachwegehahn und auch mit Hähnen der erfindungsgemäßen Ausführung Fig.3 in beliebiger Position.
